(19) Europäisches Patentamt / European Patent Office / Office européen des brevets

(11) **EP 1 925 277 A1**

(12) **DEMANDE DE BREVET EUROPEEN**

(43) Date de publication:
**28.05.2008 Bulletin 2008/22**

(21) Numéro de dépôt: **07117389.2**

(22) Date de dépôt: **27.09.2007**

(51) Int Cl.:
*A61K 8/02* *(2006.01)*       *A61K 8/891* *(2006.01)*
*A61K 8/892* *(2006.01)*       *A61Q 15/00* *(2006.01)*

(84) Etats contractants désignés:
**AT BE BG CH CY CZ DE DK EE ES FI FR GB GR HU IE IS IT LI LT LU LV MC MT NL PL PT RO SE SI SK TR**
Etats d'extension désignés:
**AL BA HR MK RS**

(30) Priorité: **21.11.2006  FR 0655029**

(71) Demandeur: **L'Oréal**
**75008 Paris (FR)**

(72) Inventeurs:
• **Lemoine, Cyril**
  **78210 Saint-cyr-l'ecole (FR)**
• **Aubrun-Sonneville, Odile**
  **92160 Antony (FR)**

(74) Mandataire: **Miszputen, Laurent**
**L'OREAL - D.I.P.I.**
**25-29 Quai Aulagnier**
**92600 Asnières (FR)**

(54) **Utilisation comme actif deodorant de particules concaves ou annulaires de materiau silicone ; procede de traitement des odeurs corporelles**

(57)     La présente invention a donc pour objet l'utilisation cosmétique de particules concaves ou annulaires de matériau siliconé, notamment sous forme de portions de sphères creuses comme agent déodorant.

Et plus particulièrement, les particules concaves de matériau siliconé utilisées sont sous forme de portions de sphères creuses ayant une section transversale en forme de fer à cheval ou d'arceau.

EP 1 925 277 A1

**Description**

La présente invention a donc pour objet l'utilisation cosmétique de particules concaves ou annulaires de matériau siliconé, notamment sous forme de portions de sphères creuses comme agent déodorant.

L'invention concerne également un procédé de traitement cosmétique de la transpiration humaine et des odeurs corporelles humaines en particulier axillaires consistant à appliquer sur la surface de la peau une quantité efficace de particules concaves ou annulaires de matériau siliconé, notamment sous forme de portions de sphères creuses comme actif déodorant.

La sueur eccrine ou apocrine est peu odorante lorsqu'elle est sécrétée. C'est sa dégradation par les bactéries via des réactions enzymatiques qui produit des composés malodorants. Les actifs déodorants ont pour fonction de diminuer ou d'empêcher la formation des mauvaises odeurs. Les différents systèmes proposés jusqu'à présent peuvent être regroupés en grandes familles.

(i) Les substances bactéricides ou limitant la croissance des bactéries :

Il y a les substances bactéricides qui détruisent la flore bactérienne résidente. Parmi ces substances, la plus employée est le Triclosan (2,4,4'-trichloro-2'-hydroxydiphényléther) qui présente l'inconvénient de modifier de façon importante l'écologie de la flore cutanée. Il y a les substances qui diminuent la croissance des bactéries. Parmi ces substances, on peut citer les chélatants de métaux de transition comme le l'EDTA ou le DPTA. Ces matériaux privent le milieu des métaux nécessaires à la croissance des bactéries. Ces actifs sont malheureusement potentiellement écotoxiques et peuvent causer des problèmes d'environnement.

(ii) Les substances bloquant les réactions enzymatiques responsables de la formation des composés odorants.

En exemples particuliers, on peut citer les inhibiteurs d'arylsulfatase, de 5-Lipoxygenase, d'aminocylase, de β-glucoronidase. Malheureusement, ces inhibiteurs sont souvent spécifiques et donc peu efficaces comparativement aux antibactériens.

(iii) Les absorbeurs des mauvaises odeurs :

Ces absorbeurs d'odeurs « capturent » ou diminuent la volatilité des composés odorants. On peut citer comme absorbeurs d'odeurs, les zéolites, les cyclodextrines. Ces composés sont difficilement formulables car les composés de la formule peuvent interagir et diminuer leur efficacité. De plus, l'absorption est souvent sélective ce qui limite l'efficacité de ces compositions.

On sait également que certains types de particules solides peuvent être utilisées comme déodorants telles que les silicates d'oxyde métallique de la demande US2005/063928 ; des particules d'oxyde métallique modifiées par un métal de transition dans les demandes US2005084464 et US2005084474, des aluminosilicates comme ceux décrits dans la demande EP1658863, des particules de dérivés de chitosan nanométriques comme celles décrites dans le brevet US6916465.

La sueur eccrine est sécrétée pour permettre la thermolyse lors de déséquilibres thermiques corporels dus à l'effort ou à la chaleur extérieure. Cette sueur est responsable des sensations d'humidité et des auréoles au niveau des vêtements. Pour éviter ces désagréments, les produits anti-transpirants ont été développés.

Les sels d'aluminium et/ou de zirconium sont les plus couramment utilisés comme actif anti-transpirant. Le principe d'action de ces actifs serait de former un gel dans le canal sudoral. Ce gel créerait un bouchon qui boucherait partiellement les pores sudoraux. Le débit sudoral est ainsi diminué. Ces sels d'aluminium ont aussi une efficacité propre car ce sont des antibactériens. Ils jouent donc aussi un rôle direct sur l'efficacité déodorante en réduisant le nombre de bactéries responsables de la dégradation de la sueur. L'utilisation de ces actifs à des concentrations élevées en vue d'obtenir une bonne efficacité entraîne des difficultés de mise en formulation. De plus, ces substances présentent un potentiel irritant pour la peau.

Il existe donc le besoin de rechercher de nouveaux actifs déodorants qui soient efficaces et facilement formulables et ne présentent pas les inconvénients des actifs déodorants connus dans l'art antérieur.

On connaît dans l'art antérieur notamment dans les demandes de brevet JP-A-2000-191789, JP-A-2003-128788, EP1579849, EP1579841, FR2867677 et FR2877839 des particules concaves de matériau d'organopolysiloxane réticulé qui ont la forme de portions de sphères creuses, obtenues par condensation de silanols résultant de l'hydrolyse de composés organosiliconés. Dans ces demandes, il est indiqué de les utiliser notamment dans des produits cosmétiques pour le visage ou dans des produits de maquillage, en particulier dans des poudres compactes de fond de teint. des sticks de rouge à lèvres. Dans les demandes de brevet EP1642619 et EP1642620, ces mêmes particules ont été proposées dans des compositions capillaires de fixation et/ou de maintien de la coiffure. On connaît également des particules siliconées de forme annulaire dans la demande de brevet US2006/0089778 pouvant être utilisées comme charge dans des produits pour le soin du visage, des produits de maquillage, des produits pour le corps, des produits capillaires ou des déodorants. L'activité déodorante de ces particules n'était pas connue dans l'enseignement de ces documents.

La demanderesse a découvert de manière inattendue et surprenante que les particules concaves ou annulaires de matériau siliconé, notamment sous forme de portions de sphères creuses présentaient en tant que telles une bonne activité déodorante et ne présentaient pas les inconvénients des autres actifs déodorants connus énumérés précédemment.

On entend au sens de la présente invention par « agent déodorant », tout composé capable de produire une diminution des mauvaises odeurs liées à la dégradation de la sueur

La présente invention a donc pour objet l'utilisation de particules concaves ou annulaires de matériau siliconé, notamment sous forme de portions de sphères creuses comme actif déodorant.

L'invention concerne également un procédé de traitement cosmétique de la transpiration humaine et des odeurs corporelles humaines en particulier axillaires consistant à appliquer une quantité efficace de particules concaves ou annulaires de matériau siliconé, notamment sous forme de portions de sphères creuses comme actif déodorant.

D'autres objets de l'invention seront décrits plus loin.

Au sens de la présente invention, on entend par "composition déodorante" toute composition capable de masquer, absorber, améliorer et/ou réduire l'odeur désagréable résultant de la décomposition de la sueur humaine par des bactéries.

Par " actif anti-transpirant", on entend toute substance ayant pour effet de diminuer ou limiter le flux sudoral.

## Particules concaves ou annulaires de matériau siliconé

Les particules concaves ou annulaires présentes dans la composition selon l'invention sont des particules siliconées, en particulier des particules de portions de sphères creuses constituées d'un matériau siliconé.

Lesdites particules ont de préférence un diamètre moyen inférieur ou égal à 10 $\mu$m, notamment allant de 0,1 $\mu$m à 8 $\mu$m, préférentiellement de 0,2 à 7 $\mu$m, plus préférentiellement allant de 0,5 à 6 $\mu$m, et de préférence encore allant de 0,5 à 4 $\mu$m.

Par diamètre moyen, on entend la plus grande dimension de la particule.

De façon avantageuse, ces particules ont une densité supérieure à 1.

Les portions de sphères creuses utilisées dans la composition selon l'invention peuvent avoir la forme de sphères creuses tronquées, présentant un seul orifice communiquant avec leur cavité centrale, et ayant une section transversale en forme de fer à cheval ou d'arceau.

Le matériau siliconé est un polysiloxane réticulé de structure tridimensionnelle ; il comprend de préférence, voire est constitué de, des motifs de formule (I) $SiO_2$ et de formule (II) $R_1SiO_{1,5}$ dans lesquels $R_1$ désigne un groupe organique ayant un atome de carbone directement relié à l'atome de silicium.

Le groupe organique $R_1$ peut être un groupe organique réactif; $R_1$ peut être plus particulièrement un groupe époxy, un groupe (méth)acryloxy, un groupe alkényle, un groupe mercaptoalkyle, aminoalkyle, halogénoalkyle, un groupe glycéroxy, un groupe uréïdo, un groupe cyano, et de préférence, un groupe époxy, un groupe (méth)acryloxy, un groupe alkényle, un groupe mercaptoalkyle, aminoalkyle. Ces groupes comprennent généralement de 2 à 6 atomes de carbone, notamment de 2 à 4 atomes de carbone.

Le groupe organique $R_1$ peut être aussi un groupe organique non réactif ; R1 peut être alors plus particulièrement un groupe alkyle en C1-C4, notamment un groupe méthyle, éthyle, propyle, butyle, ou un groupe phényle, et de préférence un groupe méthyle.

Comme groupe époxy, on peut citer un groupe 2-glycidoxyéthyle, un groupe 3-glycidoxypropyle, un groupe 2-(3,4-époxycyclohexyl)propyle

Comme groupe (méth)acryloxy, on peut citer un groupe 3-méthacryloxypropyl, un groupe 3-acryloxypropyle.

Comme groupe alkényle, on peut citer les groupes vinyle, allyle, isopropényle.

Comme groupe mercaptoalkyle, on peut citer les groupes mercaptopropyle, mercaptoéthyle.

Comme groupe aminoalkyle, on peut citer un groupe 3-(2-aminoéthyl)aminopropyle, un groupe 3-aminopropyl, un groupe N,N-diméthylaminopropyle.

Comme groupe halogénoalkyle, on peut citer un groupe 3-chloropropyle, un groupe trifluoropropyle.

Comme groupe glycéroxy, on peut citer un groupe 3-glycéroxypropyl, un groupe 2-glycéroxyéthyle.

Comme groupe uréïdo, on peut citer un groupe 2-uréidoéthyle.

Comme groupe cyano, on peut citer les groupes cyanopropyle, cyanoéthyle.

De préférence, dans le motif de formule (II), $R_1$ désigne un groupe méthyle.

Avantageusement, le matériau siliconé comprend les motifs (I) et (II) selon un rapport molaire motif (I) / motif (II) allant de 30/70 à 50/50, de préférence allant de 35/65 à 45/55.

Les particules de matériau siliconé peuvent être notamment susceptibles d'être obtenues selon un procédé comprenant :

(a) l'introduction dans un milieu aqueux, en présence d'au moins un catalyseur d'hydrolyse, et éventuellement d'au moins un tensioactif, d'un composé (III) de formule $SiX_4$ et d'un composé (IV) de formule $RSiY_3$, où X et Y désignent indépendamment l'un de l'autre un groupe alcoxy en $C_1$-$C_4$, un groupe alcoxyéthoxy renfermant un groupement alcoxy en $C_1$-$C_4$, un groupe acyloxy en $C_2$-$C_4$, un groupe N,N-dialkylamino renfermant des groupements alkyle en $C_1$-$C_4$, un groupe hydroxyle, un atome d'halogène ou un atome d'hydrogène, et R désigne un groupe organique comportant un atome de carbone directement relié à l'atome de silicium ; et

(b) la mise en contact du mélange résultant de l'étape (a) avec une solution aqueuse renfermant au moins un catalyseur de polymérisation et éventuellement au moins un tensioactif, à une température comprise entre 30 et 85°C, pendant au moins deux heures.

L'étape (a) correspond à une réaction d'hydrolyse et l'étape (b) correspond à une réaction de condensation.
Dans l'étape (a), le rapport molaire du composé (III) au composé (IV) va habituellement de 30/70 à 50/50, avantageusement de 35/65 à 45/45, et est préférentiellement de 40/60. Le rapport en poids de l'eau au total des composés (III) et (IV) va de préférence de 10/90 à 70/30. L'ordre d'introduction des composés (III) et (IV) dépend généralement de leur vitesse d'hydrolyse. La température de la réaction d'hydrolyse va généralement de 0 à 40 °C et ne dépasse habituellement pas 30°C pour éviter une condensation prématurée des composés.
Pour les groupements X et Y des composés (III) et (IV) :

Comme groupe alcoxy en $C_1$-$C_4$, on peut citer les groupes méthoxy, éthoxy ;

Comme groupe alkoxyéthoxy renfermant un groupe alcoxy en C1-C4, on peut citer les groupes méthoxyéthoxy, butoxyéthoxy ;

Comme groupe alcoxy en $C_2$-$C_4$, on peut citer les groupes acétoxy, propioxy ;

Comme groupe N,N-dilakylamino renfermant un groupement alkyle en C1-C4, on peut citer les groupes diméthylamino, diéthylamino ;

Comme atome d'halogène, on peut citer les atomes de chlore, de brome.

Comme composés de formule (III), on peut citer le tétraméthoxysilane, le tétraéthoxysilane, le tétrabutoxysilane, le triméthoxyéthoxysilane, le tributoxyéthoxysilane, le tétraacétoxysilane, le tétrapropioxysilane, le tétraacétoxysilane, le tétra(diméthylamino)silane, le tétra(diéthylamino)silane, le silane tétraol, le chlorosilane triol, le dichlorodisilanol, le tétrachlorosilane, le chlorotrihydrogénosilane. De préférence, le composé de formule (III) est choisi parmi le tétraméthoxysilane, le tétraéthoxysilane, le tétrabutoxysilane, et leurs mélanges.

Le composé de formule (III) conduit après la réaction de polymérisation à la formation des motifs de formule (I).
Le composé de formule (IV) conduit après la réaction de polymérisation à la formation des motifs de formule (II).
Le groupe R dans le composé de formule (IV) a la signification telle que décrite pour le groupe $R_1$ pour le composé de formule (II).
Comme exemples de composés de formule (IV) comportant un groupe R organique non réactif, on peut citer le méthyltriméthoxysilane, l'éthyltriéthoxysilane, le propyltributoxysilane, le butyltributoxysilane, le phényltriméthoxyéthoxysilane, le méthyl tributoxyethoxysilane, le méthyltriacétoxysilane, le méthyltripropioxysilane, le méthyltriacétoxysilane, le méthyltri(diméthylamino)silane, le méthyltri(diéthylamino)silane, le méthylsilane triol, le méthylchlorodisilanol, le méthyltrichlorosilane, le méthyltrihydrogénosilane.
Comme exemples de composés de formule (IV) comportant un groupe R organique réactif, on peut citer :

- les silanes ayant un groupe époxy comme le 3-glycidoxypropyl triméthoxysilane, le 3-glycidoxypropyl triéthoxysilane, le 2-(3,4-époxycyclohexyl)éthyl triméthoxysilane, le 3-glycidoxypropylméthyl diméthoxysilane, le 3-glycidoxypropylméthyl diméthoxysilane, le 2-glycidoxyéthylméthyldiméthoxysilane, le 3-glycidoxypropyl diméthylméthoxysilane, le 2-glycidoxyéthyl diméthylméthoxysilane ;
- les silanes ayant un groupe (méth)acryloxy comme le 3-méthacryloxypropyl triméthoxysilane, le 3-acryloxypropyl triméthoxysilane ;
- les silanes ayant un groupe alkényle comme le vinyl triméthoxysilane, l'allyl triméthoxysilane, l'isopropényl triméthoxysilane ;

- les silanes ayant un groupe mercapto comme le mercaptopropyl triméthoxysilane, le mercaptoéthyl triméthoxysilane ;
- les silanes ayant un groupe aminoalkyle comme le 3-aminopropyl triméthoxysilane, le 3-(2-aminoéthyl)aminopropyl triméthoxysilane, le N,N-diméthylaminopropyl triméthoxysilane, le N,N-diméthylaminoéthyl triméthoxy-silane ;
- les silanes ayant un groupe halogénoalkyl comme le 3-chloropropyl triméthoxysilane, le trifluoropropyl triméthoxysilane ;
- les silanes ayant un groupe glycéroxy comme le 3-glycéroxypropyl triméthoxysilane, le di(3-glycéroxypropyl) diméthoxysilane ;
- les silanes ayant un groupe uréido comme le 3-uréïdopropyl triméthoxysilane, le 3-uréïdopropyl méthyldiméthoxysilane, le 3-uréïdopropyl diméthyl-méthoxysilane ;
- les silanes ayant un groupe cyano comme le cyanopropyl triméthoxysilane, le cyanopropyl méthyldiméthoxysilane, le cyanopropyl diméthylméthoxysilane.

De préférence, le composé de formule (IV) comportant un groupe R organique réactif est choisi parmi les silanes ayant un groupe époxy, les silanes ayant un groupe (méth)acryloxy, les silanes ayant un groupe alkényle, les silanes ayant un groupe mercapto, les silanes ayant un groupe aminoalkyle.

Des exemples de composés (III) et (IV) préférés pour la mise en oeuvre de cette invention sont respectivement le tétraéthoxysilane et le méthyltriméthoxysilane.

Comme catalyseurs d'hydrolyse et de polymérisation, on peut utiliser indépendamment des catalyseurs basiques tels que l'hydroxyde de sodium, l'hydroxyde de potassium, le carbonate de sodium, l'hydrogénocarbonate de sodium, l'ammoniaque ou des amines telles que la triméthylamine, la triéthylamine, l'hydroxyde de tétraméthylammonium, ou des catalyseurs acides tels que les acides organiques comme l'acide citrique, l'acide acétique, l'acide méthanesulfonique, l'acide p-toluène sulfonique, l'acide dodécylbenzène-sulfonique, l'acide dodécylsulfonique, ou les acides minéraux tels que l'acide chlorhydrique, l'acide sulfurique, l'acide phosphorique.

Lorsqu'il est présent, le tensioactif utilisé est de préférence un tensioactif non ionique ou anionique ou un mélange des deux. Le dodécylbenzènesulfonate de sodium peut être utilisé comme tensioactif anionique. La fin de l'hydrolyse est marquée par la disparition des produits (III) et (IV), insolubles dans l'eau, et l'obtention d'une couche liquide homogène.

L'étape (b) de condensation peut utiliser le même catalyseur que l'étape d'hydrolyse ou un autre catalyseur choisi parmi ceux mentionnés précédemment.

A l'issue de ce procédé, on obtient une suspension dans l'eau de fines particules organosiliconées qui peuvent éventuellement être ensuite séparées de leur milieu. Le procédé décrit ci-dessus peut donc comprendre une étape supplémentaire de filtration, par exemple sur filtre à membrane, du produit résultant de l'étape (b), suivie éventuellement d'une étape de centrifugation du filtrat destinée à séparer les particules du milieu liquide, puis d'une étape de séchage des particules. D'autres méthodes de séparation peuvent bien entendu être employées.

La forme des portions de sphères creuses obtenues selon le procédé ci-dessus, ainsi que leurs dimensions, dépendront notamment du mode de mise en contact des produits à l'étape (b).

Un pH plutôt basique et une introduction à froid du catalyseur de polymérisation dans le mélange issu de l'étape (a) conduira à des portions de sphères creuses en forme de "bols" à fond arrondi, tandis qu'un pH plutôt acide, et une introduction goutte-à-goutte du mélange issu de l'étape (a) dans le catalyseur de polymérisation chaud, conduira à des portions de sphères creuses ayant une section transversale en forme de "fer à cheval".

Selon un mode de réalisation préféré de l'invention, on utilise des portions de sphères creuses en forme de "bols". Celles-ci peuvent être obtenues comme décrit dans la demande JP-A-2003-128788.

Des portions de sphères creuses en forme de fer à cheval sont décrites dans la demande JP-A-2000-191789.

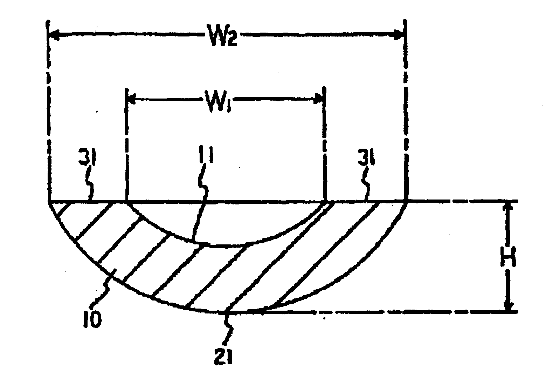

La figure 1 annexée illustre une particule concave sous forme de portions de sphères en forme de bol en coupe transversale. La largeur $W_2$ correspond au diamètre des particules.

Comme il ressort de cette figure, ces portions concaves sont formées (en coupe perpendiculaire à un plan de l'ouverture délimitée par la portion de sphère creuse) d'un petit arc interne (11), d'un grand arc externe (21) et de segments (31) qui relient les extrémités des arcs respectifs, la largeur ($W_1$) entre les deux extrémités du petit arc interne (11) allant de 0,01 à 8 $\mu$m, de préférence de 0,02 à 6 $\mu$m en moyenne, la largeur ($W_2$) entre les eux extrémités du grand arc externe (21) allant de 0,05 à 10 $\mu$m, de préférence de 0,06 à 8 $\mu$m en moyenne et la hauteur (H) du grand arc externe (21) allant de 0,015 à 8 $\mu$m, de préférence de 0,03 à 6 $\mu$m en moyenne.

Les dimensions mentionnées ci-dessus sont obtenues en calculant la moyenne des dimensions de cent particules choisies sur une image obtenue au microscope électronique à balayage.

Comme particules concaves sous forme de portions de sphères utilisables selon l'invention, on peut citer par exemple :

- les particules constituées de l'organosilicone réticulé TAK-110 (polymère réticulé méthylsilanol/silicate) de la société TAKEMOTO OIL & FAT, en forme de bol, de largeur 2,5 $\mu$m, de hauteur 1,2 $\mu$m et d'épaisseur 150 nm (particules vendues sous la dénomination NLK-506 par la société Takemoto Oil & Fat) ;

- les particules constituées de l'organosilicone réticulé TAK-110 (polymère réticulé méthylsilanol/silicate) de la société TAKEMOTO OIL & FAT, en forme de bol, de largeur 0,8 $\mu$m, de hauteur 0,4 $\mu$m et d'épaisseur 130 nm (particules vendues sous la dénomination NLK-515 par la société Takemoto Oil & Fat) ;
- les particules constituées de l'organosilicone réticulé TAK-110 (polymère réticulé méthylsilanol/silicate) de la société TAKEMOTO OIL & FAT, en forme de bol, de largeur 7 $\mu$m, de hauteur 3,5 $\mu$m et d'épaisseur 200 nm (particules vendues sous la dénomination NLK-510 par la société Takemoto Oil & Fat).

Ces particules ont pour nom INCI : Methylesilanol/silicate crosspolymer.

Avantageusement, les particules concaves siliconées ont un diamètre moyen inférieur ou égal à 5 $\mu$m, notamment allant de 0,1 $\mu$m à 5 $\mu$m, préférentiellement allant de 0,2 à 5 $\mu$m, plus préférentiellement allant de 0,5 à 4 $\mu$m, et de préférence encore allant de 0,5 à 3 $\mu$m.

Ces particules permettent, outre la réduction voire l'élimination du toucher collant, l'optimisation des propriétés de glissant, d'étalement, et de confort de la composition selon l'invention.

Les particules siliconées de forme annulaire sont de préférence choisies parmi celles décrites et synthétisées dans la demande US-A-2006/0089478. Elles présentent un diamètre moyen extérieur de 0,05 à 15 $\mu$m et un diamètre moyen intérieur de 001 à 10 $\mu$m; la différence entre le diamètre moyen extérieur et le diamètre moyen intérieur étant de 0,04 à 5 $\mu$m.

Elles présentent un réseau polysiloxane comprenant des unités siloxane de formule (1), (2) (3) (4) (5) et (6)

$$SiO_{4/2} \qquad (1)$$

$$Si(OH)_{3/2} \qquad (2)$$

$$R^1SiO_{3/2} \qquad (3)$$

$$R^2SiO_{3/2} \qquad (4)$$

$$R^3(SiOH)_{2/2} \qquad (5)$$

$$R^4(SiOH)_{2/2} \qquad (6)$$

dans lesquelles

- $R^1$ et $R^3$ désignent des groupes hydrocarbonés non réactifs, notamment des groupes alkyle, cycloalkyle, aryle, alkylaryle ou aralkyle, de préférence les groupes alkyles en $C_1$-$C_3$ notamment méthyle, éthyle, propyle, et préférentiellement un groupe méthyle
- $R^2$ et $R^4$ désignent chacun un groupe hydrocarboné choisi parmi les groupes acryloxy, methacryloxy, vinyle ou mercapto ;

le rapport molaire unités siloxane de formule (1) / unités siloxane de formule (2), (3), (4), (5) et (6) étant de 20/80 à 50/50 ;
le rapport molaire unités siloxane de formule (2), (3) et (4) / unités siloxane de formule (5) et (6) étant de 50/50 à 75/25;
le rapport molaire unités siloxane de formule (3) et (5) / unités siloxane de formule (4) et (6) étant de 20/80 à 60/40.

Comme groupe acryloxy, on peut citer un groupe 2-méthacryloxyéthyle, un groupe 3-acryloxypropyl.

Comme groupe (méth)acryloxy, on peut citer un groupe 3-méthacryloxypropyl, un groupe 3-acryloxypropyle.

Comme groupe mercaptoalkyle, on peut citer un groupe mercaptopropyl, mercaptoéthyl.

Comme groupe vinyle, on peut citer les groupes allyle, isopropenyle, 2-methylallyle.

Dans les compositions de l'invention, la concentration en particules concaves ou annulaires de matériau siliconé varie de préférence de 0,1 à 100%, plus préférentiellement de 1 à 70% et encore plus préférentiellement de 1 à 50% en poids par rapport au poids total de la composition.

Selon une forme particulière de l'invention, on utilisera les particules concaves ou annulaires de matériau siliconé comme unique actif déodorant.

Les compositions cosmétiques déodorantes selon l'invention peuvent contenir un ou plusieurs actifs déodorants additionnels comme par exemple des agents bactériostatiques ou des agents bactéricides tels que le 2,4,4'-trichloro-2'-hydroxydiphényléther (Triclosan), le 2,4-dichloro-2'-hydroxydiphényléther, le 3',4',5'-trichlorosalicylanilide, la 1-(3',4'-dichlorophenyl)-3-(4'-chlorophenyl)urée (Triclocarban) ou le 3,7,11-triméthyldodéca-2,5,10-triénol (Farnesol) ; les sels d'ammonium quaternaires comme les sels de cetyltrimethylammonium, les sels de cétylpyridinium ; la chlorhexidine et les sels; le monocaprate de diglycérol, le monolaurate de diglycérol , monolaurate de glycérol ; les sels de polyhexaméthylène biguanide.

Les compositions déodorantes selon l'invention destinées à l'usage cosmétique peuvent se présenter sous forme de lotions distribués en spray ou aérosol ; de crèmes distribuées en tubes ou en grille; de gels fluides distribués en roll-on ou en grille ; sous forme de bâtonnets (sticks) : sticks anhydres ou aqueux, sticks poudres compactés, et contenir à cet égard les ingrédients généralement utilisés dans ce type de produits et bien connus de l'homme de l'art, sous réserve qu'ils n'interfèrent pas les particules concaves ou annulaires de matériau siliconé conformes à l'invention.

Les compositions déodorantes selon l'invention destinées à l'usage cosmétique peuvent comporter au moins une phase aqueuse. Elle sont notamment formulées en lotions aqueuses ou en émulsion eau-dans-huile, huile-dans-eau, ou en émulsion multiple (émulsion triple huile-dans-eau-dans-huile ou eau-dans-huile-dans-eau (de telles émulsions sont connues et décrites par exemple par C. FOX dans « Cosmetics and Toiletries » - November 1986 - Vol 101 - pages 101-112).

La phase aqueuse desdites compositions contient de l'eau et en général d'autres solvants solubles ou miscibles dans l'eau. Les solvants solubles ou miscibles dans l'eau comprennent les mono alcools à chaîne courte par exemple en $C_1$-$C_4$ comme l'éthanol, l'isopropanol ; les diols ou les polyols comme l'éthylèneglycol, le 1,2-propylèneglycol, le 1,3-butylène glycol, l'hexylèneglycol, le diéthylèneglycol, le dipropylene glycol, le 2-éthoxyéthanol, le diéthylène glycol monométhyléther, le triéthylène glycol monométhyléther et le sorbitol. On utilisera plus particulièrement le propylèneglycol et la glycérine.

Selon une forme particulière de l'invention, les compositions déodorantes peuvent être anhydres.

Par "anhydre", on entend au sens de l'invention, une composition dont la teneur en eau libre ou ajoutée est inférieure à 3% et de préférence dont la teneur en eau ajoutée est inférieure à 1 % en poids par rapport au poids total de la composition.

Les compositions selon l'invention comprennent de préférence au moins une phase liquide organique non-miscible dans l'eau Celle-ci comprend en général un ou plusieurs composés hydrophobes qui rendent ladite phase non-miscible dans l'eau. Ladite phase est liquide (en l'absence d'agent structurant) à température ambiante (20-25 °C). De manière préférentielle, la phase organique liquide organique non-miscible dans l'eau conforme à l'invention est généralement constituée d'une huile ou de mélange d'huiles et comprend au moins 80% de composés ayant une vapeur de pression ne dépassant pas la valeur 4 kPa (30 mm Hg) à 25°C.

La phase liquide organique non-miscible dans l'eau contient de préférence une ou plusieurs huiles émollientes siliconée ou hydrocarbonée, volatiles ou non-volatiles. Ces huiles émollientes sont notamment décrites dans les brevets US 4,822,596 et US 4,904,463.

Les silicones volatiles sont définies de façon connue comme des composés volatils à température ambiante. On peut citer parmi ces composés les silicones volatiles cycliques et linéaires du type diméthylsiloxane dont les chaines comprennent de 3 à 9 résidus siliconés. De préférence on choisit les cyclométhicones $D_4$, $D_5$ ou $D_6$.

Les silicones non-volatiles sont définies de façon connue comme des composés de pression de vapeur faible à température ambiante. Parmi ces composés sont inclus: les polyalkylsiloxanes, en particulier les polyalkylsiloxanes linéaires comme par exemple les polydiméthylsiloxanes, ou diméthicones, linéaires, commercialisés par la société Dow Corning sous le nom de « Dow Corning 245 Fluid » ; les polyalkylarylsiloxanes comme par exemple les polyméthylphénylsiloxanes, commercialisés par la société Dow Corning sous le nom de « Dow Corning 556 Fluid » ; les copolymères polyéther et siloxane, comme par exemple les diméthicone copolyols.

Parmi les huiles émollientes non-volatiles utilisables dans la présente invention, on peut citer par exemple : les dérivés hydrocarbonés, les huiles minérales, les alcools gras, les esters d'alcools en $C_3$-$C_{18}$ avec des acides en $C_3$-$C_{18}$, les esters de l'acide benzoïque avec des alcools en $C_{12}$-$C_{18}$ et leurs mélanges, des polyols en $C_2$-$C_6$ choisis de préférence parmi le glycérol, le propylèneglycol ou le sorbitol, les polymères de polalkylène glycol.

Les huiles émollientes sont présentes de préférence dans des quantités allant de 1 à 50% en poids et plus préférentiellement de 5 à 40% en poids par rapport au poids total de la composition.

La composition cosmétique déodorante selon l'invention peut contenir un ou plusieurs actifs déodorants additionnels comme par exemple des agents bactériostatiques ou des agents bactéricides tels que le 2,4,4'-trichloro-2'-hydroxydiphényléther (Triclosan), le 2,4-dichloro-2'-hydroxydiphényléther, le 3',4',5'-trichlorosalicylanilide, la 1-(3',4'-dichlorophenyl)-3-(4'-chlorophenyl)urée (Triclocarban) ou le 3,7,11-triméthyldodéca-2,5,10-triénol (Farnesol) ; les sels d'ammonium quaternaires comme les sels de cetyltrimethylammonium, les sels de cétylpyridinium ; la chlorhexidine et les sels; le monocaprate de diglycérol, le monolaurate de diglycérol , monolaurate de glycérol ; les sels de polyhexaméthylène biguanide.

Afin d'améliorer l'homogénéité du produit, on peut utiliser en plus un ou plusieurs agents de suspension qui sont choisis de préférence parmi les argiles montmorillonites modifiées hydrophobes comme les bentonites ou hectorites modifiées hydrophobes. On peut citer par exemple le produit Stearalkonium Bentonite (nom CTFA) (produit de réaction de la bentonite et de l'ammonium quaternaire chlorure de stéaralkonium) tel que le produit commercial vendu sous le nom TIXOGEL MP 250 par la société Sud Chemie Rheologicals, United Catalysts Inc ou le produit Disteardimonium Hectorite (nom CTFA) (produit de réaction de l'hectorite et du chlorure de distéaryldimonium) vendu sous le nom de Bentone 38 ou Bentone Gel par la société Elementis Specialities.

Les agents de suspension sont présents de préférence dans des quantités allant de 0,1 à 5% en poids et plus préférentiellement de 0.2 à 2% en poids par rapport au poids total de la composition.

Les compositions selon l'invention peuvent également contenir en plus au moins une poudre organique.

Parmi les charges utilisables selon l'invention, on peut citer les poudres organiques. On entend dans la présente demande par « poudre organique », tout solide insoluble dans le milieu à température ambiante (25°C).

Comme poudres organiques qui peuvent être utilisées dans la composition de l'invention, on peut citer par exemple, les particules de polyamide et notamment celles vendues sous les dénominations ORGASOL par la société Atochem ; les poudres de polyéthylène ; les microsphères à base de copolymères acryliques, telles que celles en copolymère diméthacrylate d'éthylène glycol/ methacrylate de lauryle vendues par la société Dow Corning sous la dénomination de POLYTRAP ; les microsphères de polyméthacrylate de méthyle, commercialisées sous la dénomination MICROSPHERE M-100 par la société Matsumoto ou sous la dénomination COVABEAD LH85 par la société Wackherr ; les poudres de copolymère éthylène-acrylate, comme celles commercialisées sous la dénomination FLOBEADS par la société Sumitomo Seika Chemicals ; les poudres expansées telles que les microsphères creuses et notamment, les microsphères formées d'un terpolymère de chlorure de vinylidène, d'acrylonitrile et de méthacrylate et commercialisées sous la dénomination EXPANCEL par la société Kemanord Plast sous les références 551 DE 12 (granulométrie d'environ 12 $\mu$m et masse volumique 40 kg/m3), 551 DE 20 (granulométrie d'environ 30 $\mu$m et masse volumique 65 kg/m3), 551 DE 50 (granulométrie d'environ 40 $\mu$m), ou les microsphères commercialisées sous la dénomination MICROPEARL F 80 ED par la société Matsumoto ; les poudres de matériaux organiques naturels tels que les poudres d'amidon, notamment d'amidons de maïs, de blé ou de riz, réticulés ou non, telles que les poudres d'amidon réticulé par l'anhydride octénylsuccinate, commercialisées sous la dénomination DRY-FLO par la société National Starch ; les microbilles de résine de silicone telles que celles commercialisées sous la dénomination TOSPEARL par la société Toshiba Silicone, notamment TOSPEARL 240 ; les poudres d'aminoacides telles que la poudre de Lauroyl-lysine commercialisée sous la dénomination AMIHOPE LL-11 par la Société Ajinomoto ; les particules de microdispersion de cire, qui ont de préférence des dimensions moyennes inférieures à 1 $\mu$m et notamment allant de 0,02 $\mu$m à 1 $\mu$m, et qui sont constituées essentiellement d'une cire ou d'un mélange de cires, telles que les produits commercialisés sous la dénomination Aquacer par la société Byk Cera, et notamment : Aquacer 520 (mélange de cires synthétiques et naturelles), Aquacer 514 ou 513 (cire de polyéthylène), Aquacer 511 (cire polymérique), ou telles que les produits commercialisés sous la dénomination Jonwax 120 par la société Johnson Polymer (mélange de cires de polyéthylène et de paraffine) et sous la dénomination Ceraflour 961 par la société Byk Cera (cire de polyéthylène modifiée micronisée) ; et leurs mélanges.

La composition cosmétique selon l'invention peut comprendre en outre des adjuvants cosmétiques choisis parmi les cires, les adoucissants, les antioxydants, les opacifiants, les stabilisants, les agents hydratants, les vitamines, les parfums, des bactéricides, les conservateurs, les polymères, les parfums, les agents épaississants, des agents propulseurs ou tout autre ingrédient habituellement utilisé en cosmétique pour ce type d'application.

Bien entendu, l'homme de métier veillera à choisir ce ou ces éventuels composés complémentaires de manière telle que les propriétés avantageuses attachées intrinsèquement à la composition cosmétique conforme à l'invention ne soient pas, ou substantiellement pas, altérées par la ou les adjonctions envisagées.

Les cires peuvent être choisies parmi les cires animales, fossiles, végétales, minérales ou de synthèse. On peut citer notamment les cires d'abeille, les cires de Carnauba, de Candellila, de canne à sucre, du Japon, les ozokérites, la cire de Montan, les cires microcristallines, les paraffines, les cires et résines de silicone.

Les épaississants, de préférence non ioniques, peuvent être choisis parmi les gommes de guar et celluloses modifiées ou non modifiées telles que la gomme de guar hydroxypropylée, la cétylhydroxyéthylcellulose, les silices comme par exemple la Bentone Gel MIO vendue par la société NL INDUSTRIES ou la Veegum Ultra, vendue par la société POLYPLASTIC.

Les quantités de ces différents constituants pouvant être présents dans la composition cosmétique selon l'invention sont celles classiquement utilisées dans compositions déodorantes.

Les compositions selon l'invention peuvent également contenir également un ou plusieurs autres agents structurants ou gélifiants de la phase liquide organique non-miscible dans l'eau de la composition tels que les alcools gras solides linéaires et/ou les cires ; les acides gras ou leurs sels (acide stérique, stéarate de sodium, acide 12-hydroxystéarique) ; les dibenzylidene alditols (DBS) ; le lanostérol, les dérivés du N-acyl amino-acide ; les dérivés de di ou triacides carboxyliques comme les alkyl N, N'-dialkylsuccinamides (ie : dodécyl N, N'-dibutylsuccinamide) ;les organopolysiloxane élastomères tels que ceux décrits dans la demande WO97/44010.

La composition selon l'invention peut encore être pressurisée et être conditionnée dans un dispositif aérosol.

Les propulseurs généralement utilisés dans ce type de produits et bien connus de l'homme de l'art, sont comme par exemple le diméthyléther (DME) ; les hydrocarbures volatils tels que le n-butane, le propane, l'isobutane, et leurs mélanges, éventuellement avec au moins un hydrocarbure chloré et/ou fluoré; parmi ces derniers on peut citer les composés vendus par la société Dupont de Nemours sous les dénominations Fréon® et Dymel®, et en particulier le monofluorotrichlorométhane, le difluorodichlorométhane, le tétrafluorodichloroéthane et le 1,1-difluoroéthane vendu notamment sous la dénomination commerciale DYMEL 152 A par la société DUPONT. On peut également utiliser en tant qu'agent propulseur le gaz carbonique, le protoxyde d'azote, l'azote ou l'air comprimé.

La composition contenant le ou les actifs déodorants et le ou les agents propulseurs peuvent se trouver dans le même compartiment ou dans des compartiments différents dans le récipient aérosol. Selon l'invention, la concentration en agent propulseur varie généralement de 5 à 95% en poids pressurisée et plus préférentiellement de 50 à 85% en poids par rapport au poids total de la composition pressurisée.

Le moyen de distribution, qui forme une partie du dispositif aérosol, est généralement constitué par une valve de distribution commandée par une tête de distribution, elle même comprenant une buse par laquelle la composition aérosol est vaporisée. Le récipient contenant la composition pressurisée peut être opaque ou transparent. Il peut être en verre, en matériau polymérique ou en métal, recouvert éventuellement d'une couche de vernis protecteur.

L'invention concerne également un procédé de traitement cosmétique de la transpiration humaine et des odeurs corporelles humaines en particulier axillaires consistant à appliquer sur la surface de la peau une quantité efficace de particules concaves ou annulaires de matériau siliconé, notamment sous forme de portions de sphères creuses telles que définies ci-dessus comme actif déodorant et en particulier comme unique actif déodorant.

La présente invention a donc pour objet un procédé cosmétique pour traiter les odeurs axillaires humaines, consistant à appliquer sur la surface axillaire une quantité efficace des particules concaves ou annulaires de matériau siliconé on telles que définies ci-dessus comme actif déodorant et en particulier comme unique actif déodorant.

Les exemples qui suivent servent à illustrer la présente invention. Les quantités utilisées dans les compositions sont exprimées en pourcentage en poids.

### Exemples

### Exemple 1 : activité déodorante - test d'efficacité in-vitro

Des recueils de sueur axillaire sont effectués en sauna sur 6 volontaires, les échantillons de sueur individuelle, conservés dans la glace pendant quelques heures, sont alors pratiquement inodores. Ils sont ensuite mélangés pour former un pool. Le pool inbubé à 37°C pendant 24h est réparti en aliquots de 1 ml. Les actifs sont mis en contact avec ces aliquots pendant 1 heure puis mis à l'étude à 37°C pendant 5h. Après avoir laissé les échantillons revenir à température ambiante pendant 1h, un jury de 4 à 6 experts évalue l'intensité de l'odeur comparativement à un échantillon témoin : 1 ml de sueur incubée sans ajout d'actif et ayant suivi le même cycle de température. L'intensité est évaluée sur une échelle de 0 (pas d'odeur) à 4 (très forte odeur).

Les résultats sont exprimés en % de variation de l'intensité de l'odeur comparativement à cet échantillon de sueur témoin (moyenne des pourcentages de variation à T = 7h).

$$\Delta = \frac{(\text{intensité odeur de l'échantillon témoin} - \text{intensité odeur de l'échantillon avec actif})}{\text{intensité odeur de l'échantillon témoin}} \times 100$$

| Actif | % variation de l'intensité d'odeur |
|---|---|
| Silice - SB 700 (Miyoshi Kasei) (Hors invention) | -14 |
| Calcium silicate - huberderm deo (Huber) (Hors invention | -12 |
| Talc micro ACE P3 (Nippon Talc) (hors invention) | -7 |
| Microcrystalline Cellulose and Cellulose Gum (AVICEL RC-581) (hors invention) | -11 |
| Methylsilanol/silicate crosspolymer (invention) (NLK 506) | -73 |
| Methylsilanol/silicate crosspolymer (invention) (NLK-500) | -40 |
| Methylsilanol/silicate crosspolymer (invention) (NLK-505) | -42 |

Conclusion : On montre ainsi la très bonne efficacité des Particules de Takemoto, et plus particulièrement de la NLK 506.

**Exemple 2 : Stick deodorant anhydre**

| Ingrédients (nom INCI) | Quantités (% en poids) |
|---|---|
| Cyclopentasiloxane (DC 245 - Dow Corning) | 38 |
| PPG-14 Butyl ether (Ucon fluid AP- Amerchol) | 10 |
| Hydrogenated castor oil (Cutina HR -Cognis) | 4 |
| Alcool stearylique (Lorol C18 -Cognis) | 14 |
| PEG-8 distearate (Stearinerie Dubois) | 2 |
| C12-15 alkyl benzoate (Finsolv TN - Witco) | 15 |
| Methylsilanol/Silicate Crosspolymer (NLK 506 -Takemoto) | 17 |
| Total | 100 |

**Mode opératoire :**

Chauffer la cyclopentasiloxane à 65°C. Ajouter les autres ingrédients (1 par 1) en restant à 65-70°C. Homogénéiser l'ensemble (solution transparente) pendant 15 minutes. Ajouter le NLK 506. Refroidir à environ 55°C (quelques °C au dessus de l'épaississement du mélange) et couler dans les sticks. Mettre à 4°C pendant 30 minutes.

**Exemple 3 : Emulsion déodorante**

| Phase | Ingrédients (nom INCI) | Quantités (% en poids) |
|---|---|---|
| A | Methylsilanol/Silicate Crosspolymer (NLK 506 -Takemoto) | 10 |
| B | Steareth-21 (Brij 721- ICI) | 2 |
| | Steareth-2 (Brij 72- ICI) | 2 |
| | PPG-15 stearyl ether (Arlamol E - ICI) | 1,5 |
| | Cyclopentasiloxane (DC 245 - Dow Corning) | 3,5 |
| C | Eau | qsp 100 |

**Mode opératoire :**

On chauffe les phases (B) et (C) séparément à 70°C. On mélange les phases (B) et (C) sous agitation Turax 5min puis on refroidit à 55°C sous pâle. On ajoute la phase A doucement en agitant. On homogénéise pendant 1 à 3min. On refroidit à 35°C sous agitation.

**Exemple 4 : Emulsion déodorante et antitranspirante (roll'on)**

| Phase | Ingrédients (nom INCI) | Quantités (% en poids) |
|---|---|---|
| A | Aluminium Chlorhydrate (50%solution) (Chlorhydrol 50% - USP) | 35 |
| | Methylsilanol/Silicate Crosspolymer (NLK 506 -Takemoto) | 6 |

(suite)

| Phase | Ingrédients (nom INCI) | Quantités (% en poids) |
|---|---|---|
| B | Steareth-21 (Brij 721- ICI) | 2 |
| | Steareth-2 (Brij 72- ICI) | 2 |
| | PPG-15 stearyl ether (Arlamol E - ICI) | 1,5 |
| | Cyclopentasiloxane (DC 245 - Dow Corning) | 3,5 |
| C | Eau | qsp 100 |

## Mode opératoire :

On chauffe les phases (B) et (C) séparément à 70°C. On mélange les phases (B) et (C) sous agitation Turax 5min puis on refroidit à 55°C sous pâle. On ajoute la phase A doucement en agitant. On homogénéise pendant 1 à 3min. On refroidit à 35°C sous agitation.

## Exemple 5 : Pâte déodorante

| Ingrédients (nom INCI) | Quantités (% en poids) |
|---|---|
| Triethyl Citrate (CITROFLEX 2- REILLY CHEMICALS) | 7 |
| Isopropylpalmitate (COGNIS) | 6 |
| Methylsilanol/Silicate Crosspolymer (NLK 506 -Takemoto) | 17,5 |
| Cyclomethicone (DOW CORNING 245 FLUID) | 60.5 |
| Cyclopentasiloxane (and) Dimethiconol (DOW CORNING 1501 FLUID) | 9 |

## Mode opératoire :

On disperse les particules d Methylsilanol/Silicate dans le mélange des autres matières premières à la pale. On obtient une pâte homogène.

## Exemple 6 : Aérosol

| Phase | Ingrédients (nom INCI) | Quantités (% en poids) |
|---|---|---|
| A | Triethyl Citrate (CITROFLEX 2-REILLY CHEMICALS) | 1,05 |
| | Stearalkonium Bentonite | 0,23 |
| | Isopropylpalmitate (COGNIS) | 0,9 |
| | Methylsilanol/Silicate Crosspolymer (NLK 506 -Takemoto) | 2,625 |
| | Cyclomethicone (DOW CORNING 245 FLUID) | 9,075 |
| | Cyclopentasiloxane (and) Dimethiconol (DOW CORNING 1501 FLUID) | 1,35 |
| B | Isobutane | 85 |

## Mode opératoire :

On disperse les particules de Methylsilanol/Silicate dans le mélange des autres matières premières à la pale consituant la phase A. On pressurise dans un bidon aérosol avec de l'isobutane.

**Revendications**

1. Utilisation cosmétique de particules concaves ou annulaires de matériau siliconé, notamment sous forme de portions de sphères creuses comme actif déodorant.

2. Utilisation selon la revendication 1, **caractérisée en ce que** les particules concaves de matériau siliconé ont un diamètre moyen inférieur ou égal à 10 μm.

3. Utilisation selon l'une quelconque des revendications 1 à 3, **caractérisée en ce que** les particules concaves de matériau siliconé sont sous forme de portions de sphères creuses ayant une section transversale en forme de fer à cheval ou d'arceau.

4. Utilisation selon l'une quelconque des revendications précédentes, **caractérisée en ce que** le matériau siliconé est un polysiloxane réticulé de structure tridimensionnelle comprenant, ou constitué de, des motifs de formule (I) : $SiO_2$ et de formule (II) : $R_1SiO_{1,5}$ dans lesquelles $R_1$ désigne un groupe organique ayant un atome de carbone directement relié à l'atome de silicium.

5. Utilisation selon la revendication 4, **caractérisée en ce que** $R_1$ est choisi parmi les groupes alkyle en $C_1$-$C_4$, phényle, époxy, (méth)acryloxy, alkényle, mercaptoalkyle, aminoalkyle, halogénoalkyle, glycéroxy, uréido, cyano.

6. Utilisation selon l'une quelconque des revendications 4 à 6, **caractérisée en ce que** le matériau siliconé comprend les motifs (I) et (II) selon un rapport molaire motif (I) / motif (II) allant de 30/70 à 50/50, de préférence allant de 35/65 à 45/55.

7. Utilisation selon l'une quelconque des revendications précédentes, **caractérisée en ce que** les particules concaves sont formées d'un petit arc interne (11), d'un grand arc externe (21) et de segments (31) qui relient les extrémités des arcs respectifs, la largeur (W1) entre les deux extrémités du petit arc interne (11) allant de 0,01 à 8 μm, de préférence de 0,02 à 6 μm en moyenne, la largeur (W2) entre les eux extrémités du grand arc externe (21) allant de 0,05 à 10 μm, de préférence de 0,06 à 8 μm en moyenne et la hauteur (H) du grand arc externe (21) allant de 0,015 à 8 μm, de préférence de 0,03 à 6 μm en moyenne.

8. Utilisation selon la revendication 1, **caractérisée en ce que** les particules de forme annulaire présentent un diamètre moyen extérieur de 0,05 à 15 μm et un diamètre moyen intérieur de 001 à 10 μm; la différence entre le diamètre moyen extérieur et le diamètre moyen intérieur étant de 0,04 à 5μm.

9. Utilisation selon la revendication 1 ou 2, où les particules annulaires de matériau siliconé présentent un réseau polysiloxane comprenant des unités siloxane de formule (1), (2) (3) (4) (5) et (6)

$$SiO_{4/2} \qquad (1)$$

$$Si(OH)_{3/2} \qquad (2)$$

$$R^1SiO_{3/2} \qquad (3)$$

$$R^2SiO_{3/2} \qquad (4)$$

$$R^3(SiOH)_{2/2} \qquad (5)$$

$$R^4(SiOH)_{2/2} \qquad (6)$$

dans lesquelles $R^1$ et $R^3$ désignent des groupes alkyle, cycloalkyle, aryle, alkylaryle ou aralkyle, et $R^2$ et $R^4$ désignent chacun un groupe hydrocarboné choisi parmi les groupes acryloxy, methacryloxy, vinyle ou mercapto ;
le rapport molaire unités siloxane de formule (1) / unités siloxane de formule (2), (3), (4), (5) et (6) étant de 20/80 à 50/50 ;
le rapport molaire unités siloxane de formule (2), (3) et (4) / unités siloxane de formule 5) et (6) étant de 50/50 à 75/25;
le rapport molaire unités siloxane de formule (3) et (5) / unités siloxane de formule (4) et (6) étant de 20/80 à 60/40.

10. Utilisation selon l'une quelconque des revendications précédentes, où les particules concaves ou annulaires de matériau siliconé sont utilisées comme unique actif déodorant.

11. Procédé cosmétique pour traiter les odeurs axillaires humaines, consistant à appliquer sur la surface axillaire une quantité efficace des particules concaves ou annulaires de matériau siliconé telles que définies selon l'une quelconque des revendications précédentes comme actif déodorant.

12. Procédé selon la revendication 11, où lesdits particules sont utilisées comme unique actif déodorant.

Office européen
des brevets

**RAPPORT DE RECHERCHE EUROPEENNE**

Numéro de la demande

EP 07 11 7389

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X | JP 2003 128788 A (TAKEMOTO OIL & FAT CO LTD) 8 mai 2003 (2003-05-08) * abrégé * | 1-12 | INV. A61K8/02 A61K8/891 A61K8/892 |
| X | JP 2000 191789 A (TAKEMOTO OIL & FAT CO LTD) 11 juillet 2000 (2000-07-11) * abrégé * | 1-12 | A61Q15/00 |
| Y | US 2005/220741 A1 (DUMOUSSEAUX CHRISTOPHE) 6 octobre 2005 (2005-10-06) * alinéa [0002] - alinéa [0004] * * alinéa [0014] - alinéa [0056] * | 1-12 | |
| Y | WO 02/50166 A (DOW CORNING) 27 juin 2002 (2002-06-27) * alinéa [0013] - alinéa [0044] * * alinéa [0078] - alinéa [0079] * | 1-12 | |
| Y | US 2002/037974 A1 (GENERAL ELECTRIC CO.) 28 mars 2002 (2002-03-28) * alinéa [0024] - alinéa [0084] * * revendication 23; exemples 14,15 * | 1-12 | DOMAINES TECHNIQUES RECHERCHES (IPC) |
| A | US 2005/249763 A1 (L'OREAL) 10 novembre 2005 (2005-11-10) * alinéa [0090] * * alinéa [0182] - alinéa [0183] * | 1-12 | A61K |
| A | EP 1 579 841 A (L'OREAL) 28 septembre 2005 (2005-09-28) * alinéa [0001] - alinéa [0008] * * alinéa [0043]; revendication 59 * | 1-12 | |

Le présent rapport a été établi pour toutes les revendications

| Lieu de la recherche | Date d'achèvement de la recherche | Examinateur |
|---|---|---|
| Munich | 18 mars 2008 | Irwin, Lucy |

EPO FORM 1503 03.82 (P04C02)

**ANNEXE AU RAPPORT DE RECHERCHE EUROPEENNE
RELATIF A LA DEMANDE DE BREVET EUROPEEN NO.**

EP 07 11 7389

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

18-03-2008

| Document brevet cité au rapport de recherche | | Date de publication | Membre(s) de la famille de brevet(s) | | Date de publication |
|---|---|---|---|---|---|
| JP 2003128788 | A | 08-05-2003 | JP | 3701016 B2 | 28-09-2005 |
| JP 2000191789 | A | 11-07-2000 | AUCUN | | |
| US 2005220741 | A1 | 06-10-2005 | AUCUN | | |
| WO 0250166 | A | 27-06-2002 | AU | 4164302 A | 01-07-2002 |
| | | | EP | 1343834 A2 | 17-09-2003 |
| | | | JP | 2004526001 T | 26-08-2004 |
| | | | US | 2003050393 A1 | 13-03-2003 |
| US 2002037974 | A1 | 28-03-2002 | AUCUN | | |
| US 2005249763 | A1 | 10-11-2005 | AUCUN | | |
| EP 1579841 | A | 28-09-2005 | FR | 2867676 A1 | 23-09-2005 |
| | | | JP | 2005272466 A | 06-10-2005 |

EPO FORM P0460

Pour tout renseignement concernant cette annexe : voir Journal Officiel de l'Office européen des brevets, No.12/82

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 2005063928 A
- US 2005084464 A
- US 2005084474 A
- EP 1658863 A
- US 6916465 B
- JP 2000191789 A
- JP 2003128788 A
- EP 1579849 A
- EP 1579841 A
- FR 2867677
- FR 2877839
- EP 1642619 A
- EP 1642620 A
- US 20060089778 A
- US 20060089478 A
- US 4822596 A
- US 4904463 A
- WO 9744010 A

**Littérature non-brevet citée dans la description**

- **C. FOX.** *Cosmetics and Toiletries,* Novembre 1986, vol. 101, 101-112